# EUROPÄISCHE PATENTSCHRIFT

(11) **EP 2 988 707 B1**
(45) Veröffentlichungstag und Bekanntmachung des Hinweises auf die Patenterteilung: **07.06.2017**
(21) Anmeldenummer: 14719241.3
(22) Anmeldetag: 17.04.2014
(51) Int. Cl.: A61F 5/01

(54) **ENTLASTUNGSORTHESE**
RELIEVING ORTHOSIS
ORTHÈSE DE DÉCHARGE

(30) Priorität: 22.04.2013 DE 102013207256
(43) Veröffentlichungstag der Anmeldung: 02.03.2016
(73) Patentinhaber: Otto Bock HealthCare GmbH, 37115 Duderstadt (DE)
(72) Erfinder: LIDOLT, Klaus, 37115 Duderstadt (DE); LJUBIMIR, Boris, 8010 Graz (AT); VOLLBRECHT, Matthias, 37412 Herzberg (DE); JOHANNES, Klaus, 37130 Gleichen (DE); CHRISTENHUSZ, Harry, 48455 Bad Bentheim (DE)
(74) Vertreter: Stornebel, Kai
(86) Internationale Anmeldenummer: PCT/EP2014/001035
(87) Internationale Veröffentlichungsnummer: WO 2014/173512

(56) Entgegenhaltungen:
- EP-A1- 0 647 440
- EP-A1- 0 955 023
- DE-U1- 9 205 681
- DE-U1- 29 924 933
- US-A- 5 088 479
- US-B1- 7 666 155

## Beschreibung

Die Erfindung betrifft eine Entlastungsorthese mit einem Fußteil, das eine Sohle aufweist, einem sich in proximaler Richtung von dem Fußteil weg erstreckenden Unterschenkelteil sowie zumindest einem Befestigungselement zur Festlegung der Orthese an einem Nutzer, die an dem Fußteil und/oder dem Unterschenkelteil angeordnet sind, wobei das Fußteil längenveränderbar ausgebildet ist. Eine solche Entlastungsorthese ist insbesondere als Fersenentlastungsorthese bei Kalkaneusfrakturen einsetzbar.

Fersenentlastungsorthesen aus dem Stand der Technik sind beispielsweise durch die 28F10 der Firma Otto Bock HealthCare GmbH bekannt, mit der eine frühfunktionale Therapie von Kalkaneusfrakturen möglich ist. Die Orthese entlastet das Fersenbein und unterstützt das Längsgewölbe sowie den Unterschenkelbereich. Durch auswechselbare Einsätze ist es möglich, sukzessive eine höhere Gewichtsbelastung auf das Fersenbein auszuüben.

Die EP 0 647 440 A1, die DE 92 05 681 U1 und die US 7,666,155 B1 betreffen jeweils Entlastungsorthesen gemäß dem Oberbegriff von Anspruch 1.

Die EP 0 955 023 A1 betrifft eine Orthese mit einem zumindest einen unteren Teil des Unterschenkels wenigstens teilweise umschließenden Schaft und einen über ein schalenförmig ausgebildetes Fersenteil mit dem Schaft verbundenes Fußteil. Zwischen einer Spitze des Fußteils und dem Fersenteil ist eine die Orthese nach unten abschließende durchgehende Lauffläche angeordnet. Eine Längenverstellung des Fußteils, des Fersenteils oder der Lauffläche ist nicht möglich.
Aufgabe der vorliegenden Erfindung ist es, eine Entlastungsorthese bereitzustellen, mit der eine natürliche Gangbewegung des Nutzers möglich ist.

Erfindungsgemäß wird diese Aufgabe durch eine Entlastungsorthese mit den Merkmalen des Hauptanspruches gelöst, vorteilhafte Ausgestaltungen der Erfindung sind in den Unteransprüchen, der Beschreibung und den Figuren offenbart.

Die Entlastungsorthese mit einem Fußteil, das eine Sohle aufweist, einen sich in proximaler Richtung von dem Fußteil weg erstreckenden Unterschenkelteil sowie zumindest einem Befestigungselement zur Festlegung der Orthese an einen Nutzer, das an dem Fußteil und/oder dem Unterschenkelteil angeordnet ist, wobei das Fußteil längenveränderbar ausgebildet ist, sieht vor, dass das Fußteil eine Vorderfußkomponente aufweist, die biegeelastisch ausgebildet ist. Durch die Längenveränderbarkeit des Fußteils ist es möglich, eine exakte Anpassung der Orthese an die Fußlänge oder Fußbreite und damit an den jeweiligen Patienten vorzunehmen, ohne dass für jede Fußgröße eine gesonderte Orthese angefertigt werden oder ein Patient eine nicht exakt passende Orthese tragen muss. Dadurch, dass die Vorderfußkomponente biegeelastisch ausgebildet ist, wird ein Abrollen ermöglicht.

Eine Weiterbildung der Erfindung sieht vor, dass ein Längsgewölbeformteil vorgesehen ist, das an dem Fußteil befestigt ist und ein Fersenbein beim Gehen oder Stehen entlastet. Über das Längsgewölbeformteil wird erreicht, dass bei einer Kalkaneusfraktur zunächst das Fersenbein komplett entlastet wird und die auftretenden Kräfte über das Fußgewölbe, die Sohle und das Unterschenkelteil abgeleitet werden, so dass insbesondere nach frischen Frakturen zunächst keine Belastung oder nur eine sehr geringe Belastung auf das Fersenbein ausgeübt werden kann.

Das Fußteil kann mehrteilig ausgebildet sein und neben der Vorderfußkomponente eine Fersenkomponente aufweisen, die verschieblich zueinander und aneinander festlegbar ausgebildet sind. Dadurch ist es möglich, dass die Vorderfußkomponente relativ zu der Fersenkomponente verlagerbar ist und in der jeweils gewünschten Position zueinander fixiert werden kann. Es ist auch vorgesehen, dass die Länge auch in Quererstreckung veränderbar ist, so dass die Breite der Entlastungsorthese eingestellt werden kann.

Die Vorderfußkomponente und die Fersenkomponente können in einer Längsführung aneinander gelagert sein, beispielsweise in einer Schienen- und Nutführung, und über Klemmelemente oder Formschlusselemente zueinander fixierbar sein. Dadurch ist es möglich, durch einfaches Lösen oder Entfernen der Klemmelemente oder Formschlusselemente die Längsverschieblichkeit der beiden Fußteilkomponenten zueinander herzustellen und nach Positionierung der Komponenten zueinander diese in der gewünschten Stellung zu verriegeln. Die Längsführung erleichtert dabei die Verlagerung, da eine grundsätzliche Zuordnung und Orientierung der Vorderfußkomponente zu der Fersenkomponente weiterhin erhalten bleibt.

In oder an der Vorderfußkomponente und der Fersenkomponente können Rastelemente angeordnet oder ausgebildet sein, um die beiden Komponenten zueinander auszurichten. Ebenfalls ist es möglich, dass an der Vorderfußkomponente und/oder der Fersenkomponente Ausrichtmarkierungen vorhanden sind, über die es möglich ist, die Komponenten zueinander auszurichten, die Länge der Orthese einzustellen und dann durch Klemmelemente, Formschlusselemente oder andere Einrichtungen zueinander zu fixieren.

Die Befestigungselemente ihrerseits, mit denen die Orthese an dem Nutzer festlegbar ist, können verlagerbar an dem Fußteil und/oder dem Unterschenkelteil festgelegt sein, so dass die Individualisierung der Orthese weiter verbessert werden kann, indem die Befestigungselemente an denjenigen Orten an der Orthese positioniert werden, an denen eine bestmögliche Festlegung der Orthese bei gleichzeitig geringstmöglicher Beeinträchtigung und bei maximalem Komfort für den Orthesennutzer gewährleistet sind.

Die Befestigungselemente können an Langlöchern oder an in diskreten zueinander angeordneten Ausnehmungen oder Halteeinrichtungen festgelegt sein. Die Ausnehmungen oder Halteeinrichtungen legen denjenigen Bereich fest, entlang der die Befestigungselemente an dem Fußteil und/oder Unterschenkelteil festlegbar sind. Langlöcher ermöglichen eine stufenlose Verschiebung der Befestigungselemente und damit eine autoadaptive Ausrichtung an den jeweiligen Nutzer.

Das Längsgewölbeformteil kann lösbar oder verlagerbar an dem Fußteil befestigt sein, beispielsweise über Klettverschlussverbindungen, eine Längsführung oder in Abständen zueinander angeordnete Bohrungen oder Gewinde, so dass eine Festlegung in diskreten Abständen oder quasi kontinuierlich verlagerbar erreicht werden kann.

Die Klettverschlusselemente können in mehreren Positionen an dem Fußteil festlegbar ausgestaltet sein, wobei die Klettverschlusselemente insbesondere dazu vorgesehen sind, das Längsgewölbeformteil an dem Fußteil festzulegen. Dazu ist es vorgesehen, dass die Klettverschlusselemente einen Teil der Haken- und Flauschkomponenten eines Klettverschlusses ausbilden, so dass das Längsgewölbeformteil nutzerindividuell an dem Fußteil festlegbar ist.

Die Befestigungselemente zum Befestigen der Orthese an dem Fuß können als Y-Klettverbindung ausgebildet sein, um eine leichte Längenanpassbarkeit erreichen zu können. Das Y-Klettelement ist vorteilhafterweise an einer Oberfläche und den einander gegenüberliegenden Innenseiten als Hakenelemente ausgebildet, während der Rest des Befestigungselementes als Flauschelement ausgebildet ist, so dass durch Kürzen des Flauschelementes und Festlegen des Y-Klettelementes an dem Flauschelement eine Längenanpassbarkeit erreicht werden kann. Zur Festlegung der Orthese an dem Fuß bzw. Unterschenkel ist das Befestigungselement durch einen Schlitz oder ein Umlenkelement hindurchführbar und durch Umschlagen auf sich zurück fixierbar.

An der Außenseite des Fußteils, entweder an der Vorderfußkomponente oder an der Fersenkomponente, können Markierungen für Schuhgrößen angebracht sein, so dass eine grobe Voreinstellung anhand der grundsätzlich bekannten Schuhgröße des Orthesennutzers erfolgen kann. Ebenfalls können Markierungen an dem Fußteil für die Position des Längsgewölbeformteils in Abhängigkeit von den üblichen Schuhgrößen angebracht sein, so dass ein Voreinstellung und eine leichtere Positionierbarkeit des Längsgewölbeformteils erfolgen kann.

Vorteilhafterweise ist die Entlastungsorthese mit dem Fußteil und dem Unterschenkelteil in Längsrichtung symmetrisch aufgebaut, so dass eine Anwendung sowohl für den rechten Fuß als auch für den linken Fuß möglich ist. Die Fersenkomponente dient dabei als sogenannter Container, der die Ferse in vier Richtungen einschließt. In oder an dem Längsgewölbeformteil kann ein Röntgenpunkt angeordnet sein, um die physiologisch exakte Positionierung des Längsgewölbeformteils innerhalb der Entlastungsorthese vornehmen zu können. Durch den Röntgenpunkt, der eine vergleichsweise hohe Dichte aufweist und beispielsweise aus Metall ausgebildet sein kann, kann nach dem ersten Anlegen der Orthese durch eine Röntgenaufnahme überprüft werden, ob das Längsgewölbeformteil richtig positioniert ist.

Nachfolgend werden Ausführungsbeispiele der vorliegenden Erfindung anhand der beigefügten Figuren näher erläutert. Es zeigen:
- Figur 1 -: eine perspektivische Gesamtansicht einer Entlastungsorthese;
- Figur 2 -: eine Explosionsdarstellung einer ersten Ausführungsform;
- Figur 3 -: eine Explosionsdarstellung einer zweiten Ausführungsform;
- Figur 4 -: Untenansichten einer Entlastungsorthese in unterschiedlichen Längen;
- Figur 5 -: eine Explosionsdarstellung der Variante gemäß Figur 2 in Schräg-draufsicht; sowie
- Figur 6 -: eine perspektivische Ansicht einer weiteren Variante.

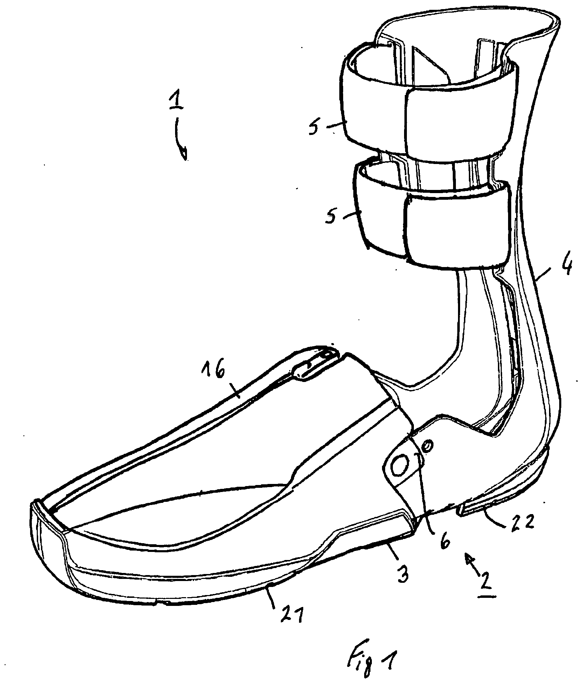

Figur 1 zeigt eine Entlastungsorthese 1 in Gestalt einer Fersenentlastungsorthese mit einem Fußteil 2, das eine Sohle 3 aufweist. An die Sohle 3 schließt sich an dem hinteren Ende ein Unterschenkelteil 4 an, das sich von dem Fußteil 2 in proximaler Richtung erstreckt. Sowohl an dem Fußteil 2 als auch an dem Fersenteil 4 sind Befestigungselemente 5, 6 angeordnet, um die Entlastungsorthese 1 an einem nicht dargestellten Nutzer anzulegen. Die Befestigungselemente 5 an dem Fersenteil 4 sind in Gestalt von Klettverschlussriemen ausgebildet. Die Befestigungselemente 5 schließen das posterior geschlossene Fersenteil 4 in anteriorer Richtung.

An dem Fußteil 2 ist ein über den Fußrücken führendes Befestigungselement 6 in Gestalt eines Klettverschlussriemens befestigt. Die Befestigungselemente 5, 6 an dem Fersenteil 4 und dem Fußteil 2 werden, nachdem sie durch eine Führung, einen D-Ring, eine Lasche oder einen Schlitz geführt wurden, auf sich selbst zurückgeschlagen und festgelegt, um die Entlastungsorthese 1 an dem Fuß zu befestigen.

Das Fußteil 2 mit der Sohle 3 ist zweiteilig ausgebildet und weist eine Vorder-fußkomponente 21 und eine Fersenkomponente 22 auf. Die Vorderfußkomponente 21 ist verlagerbar an der Fersenkomponente 22 befestigt. Von der Fersenkomponente 22 erstreckt sich das Unterschenkelteil 4 einstückig in proximaler Richtung. Neben der einstückigen Ausgestaltung des Unterschenkelteils 4 mit der Fersenkomponente 22 ist es möglich, das Unterschenkelteil 4 und die Fersenkomponente 22 als separate, aneinander festgelegte Bauteile auszubilden.

An der Vorderfußkomponente 21 ist ein Schutzüberzug 16 angeordnet, der verschließbar ausgebildet ist, um im angelegten Zustand der Entlastungsorthese den Fuß zu umgeben und eine schuhähnliche Anmutung zu erreichen.

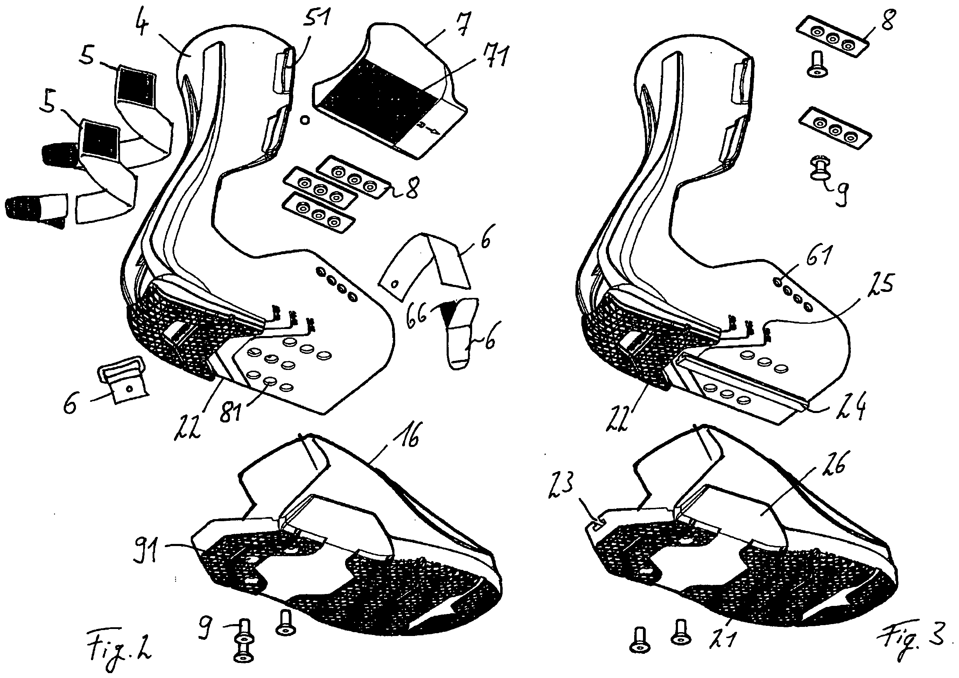

Figur 2 zeigt eine erste Variante der Entlastungsorthese 1 in einer Explosions-darstellung von schräg unten. Die einzelnen Elemente der Entlastungsorthese 1 sind dargestellt. Neben der einstückigen Ausgestaltung der Fersenkomponente 22 mit dem Unterschenkelteil 4 sind die Befestigungselemente 5, 6 in Gestalt eines Y-Klettverschlusses dargestellt. Die Befestigungselemente 5 an dem Unterschenkelteil 4 sind über Langlöcher 51, die medial und lateral an dem proximalen Ende des Unterschenkelteils 4 angeordnet sind, geführt. Über die Langlöcher 51 ist es möglich, einerseits eine Verschieblichkeit in proximal-distal-Richtung zu erreichen und andererseits das freie Ende des Befestigungselementes 5 durchzuführen, auf sich selbst zurück umzulegen und an sich zu befestigen. Dazu ist es vorgesehen, dass ein Teil des Befestigungselementes 5 als Flauschriemen ausgebildet ist, an dessen Ende ein Y-Stück angeordnet ist, das an zumindest einer Seite Hakenkomponenten aufweist, so dass nach dem Durchführen des Y-Stückes durch ein Langloch 51 und dem Zurückschlagen auf den Flauschriemen eine Festlegung an dem Flauschriemen erreicht werden kann.

An der Fersenkomponente 22 sind Ausnehmungen 61 nebeneinander angeordnet, die sich in anterior-posterior-Richtung erstrecken, so dass das Befestigungselement 6, das ebenfalls als Y-Klettriemen ausgebildet ist, an der jeweils gewünschten Position befestigbar ist. Ein D-Schnallenstück ist als Gegenstück vorgesehen, das an einer kor-respondierenden Ausnehmung 61 an dem oberen Rand des schalenartig ausgebildeten Teils der Fersenkomponente 22 anordbar ist.

An dem Sohlenbereich der Fersenkomponente 22 sind im dargestellten Ausführungsbeispiel drei in anterior-posterior-Richtung orientierte Löcher 81 in drei nebeneinander angeordneten Reihen ausgebildet, die zur Aufnahme von kor-respondierenden Vorsprüngen in Klettverschlusselementen 8 vorgesehen sind. In den Vorsprüngen an den Klettverschlusselementen 8 sind Gewinde eingebracht, um Schrauben 9 durch Ausnehmungen 91 und die Löcher 81 hindurch einzuschrauben und somit die Vorderfußkomponente 21 in einer der drei möglichen Positionen festzulegen. An der Fersenkomponente 22 sind an der Außenseite Markierungen 25 angeordnet, die anzeigen, in welcher Größeneinstellung die Vorderfußkomponente 21 an der Fersenkomponente 22 festgelegt ist.

Auf der zum Nutzer hin gerichteten Oberseite ist das Klettverschlusselement 8 jeweils mit Flausch oder Haken eines Klettverschlusses 82 versehen, um mit dem korrespondierenden Klettverschlusselement 71, das auf der Unterseite eines Längsgewölbeformteils 7 angeordnet ist, zusammenzuwirken. Das Längsgewölbeformteil 7 kann in der gewünschten Position in anterior-posterior-Richtung auf die Klettverschlusselemente 8 aufgesetzt und örtlich fixiert werden. Dadurch ist es möglich, eine veränderliche, in der gewählten Position jedoch feste Zuordnung des Längsgewölbeformteils 7 zu dem Unterschenkelteil 4, der Fersenkomponente 22 und der Vorderfußkomponente 21 zu erreichen.

Figur 3 zeigt in der gleichen Perspektive wie in der Figur 2 eine Variante der Erfindung, bei der statt dreier Klettverschlusselemente 8 nur zwei angeordnet sind. An der Unterseite der Fersenkomponente 22 ist eine Führungsschiene 24 mit einem T-förmigen Querschnitt angeordnet. An der Oberseite der Vorderfußkomponente 21 ist eine korrespondierende Nut 23 eingearbeitet, so dass die Vorderfußkomponente 21 entlang einer Längsführung, die durch die T-förmige Schiene 24 und die korrespondierende Nut 23 ausgebildet ist, in anterior-posterior-Richtung verlagerbar ist. Über die Schrauben 9 kann eine Festlegung der Vorderfußkomponente 21 an der Fersenkomponente 22 entweder formschlüssig oder klemmend erfolgen. Andere Fixierungsmöglichkeiten sind ebenfalls möglich. Über die Markierungen 25 kann eine Größeneinstellung erfolgen, die Markierungen 25 werden je nach Ausrichtung der Vorderfußkomponente 21 zur Fersenkomponente 22 durch einen Absatz 26 verdeckt oder freigegeben.

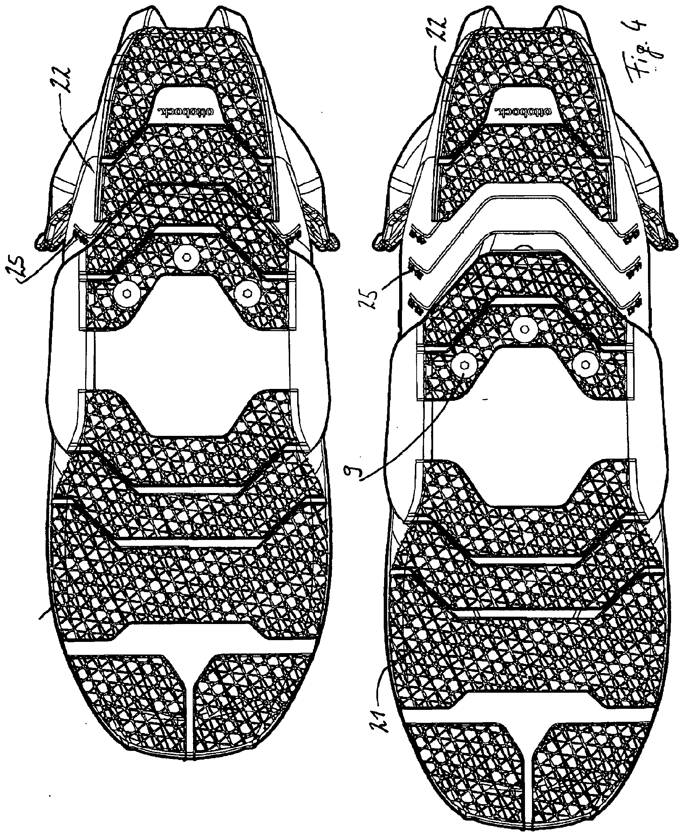

Figur 4 zeigt eine Entlastungsorthese 1 in Unteransicht in verschiedenen Längeneinstellungen. Die obere Darstellung zeigt die Vorderfußkomponente 21 und die Fersenkomponente 22 in maximal angenäherter Position, also in der kürzesten Version der Entlastungsorthese 1. Nur eine Markierung 25 ist zu erkennen. In der unteren Darstellung sind die Schrauben 9 in den vordersten Gewinden der Klettverschlusselemente 8 eingeschraubt, so dass drei Markierungen 25 zu erkennen sind, was bedeutet, dass die längste Version der Entlastungsorthese 1 eingestellt ist.

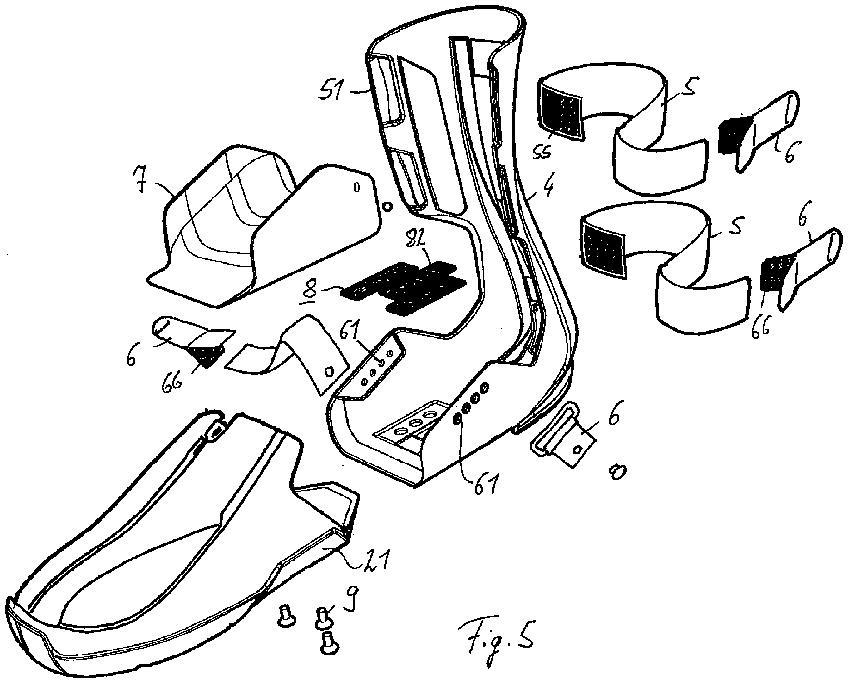

In der Figur 5 ist die Entlastungsorthese 1 gemäß der Figuren 1 und 2 in anderer perspektivischer Darstellung gezeigt. Die Einzelkomponenten entsprechen denen der Figuren 1 und 2, jedoch sind sowohl die Langlöcher 51 als auch die Ausgestaltungen der Befestigungselemente 5, 6 als Y-Klettverschlussbänder besser zu erkennen. Ebenso können die Klettverschlusselemente 8 mit den Flausch- oder Hakenelementen auf der Oberseite zur einstellbaren Festlegung des Längsgewölbeformteils 7 an der Orthese besser erkannt werden. Durch die Ausnehmungen in den Befestigungselementen 6 über den Fußrücken kann über Stopfen, Schrauben oder dergleichen, eine formschlüssige Festlegung an den Löchern 61 in der Fersenkomponente 21 erfolgen.

Auf der Unterseite des Fußteils 2 können Profilelemente zum verbesserten Halt auf den Untergrund angebracht sein. Die Vorderfußkomponente 21 ist erfindungsgemäß biegeelastisch ausgebildet, um ein Abrollen zu ermöglichen.

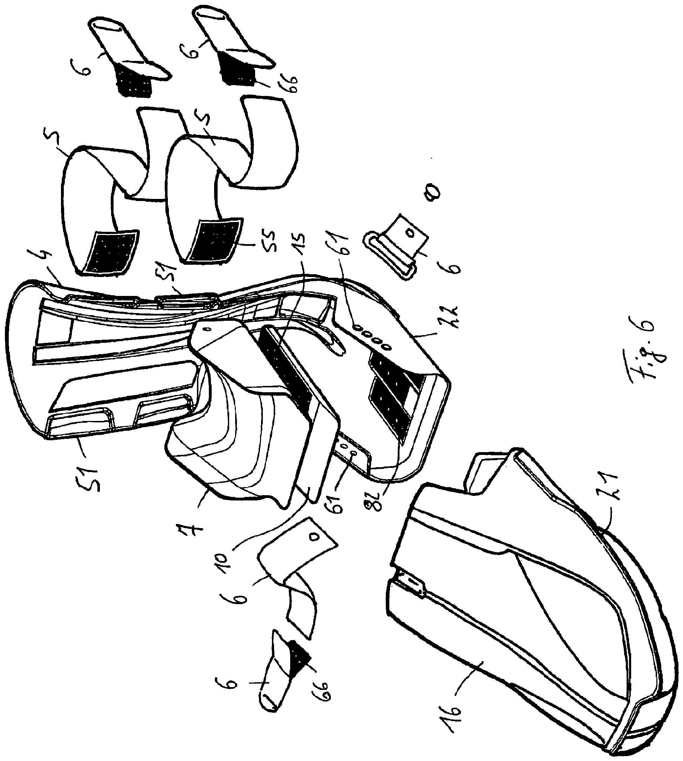

In der Figur 6 ist eine Variante der Erfindung gezeigt, bei der statt einer direkten Befestigung des Längsgewölbeformteils 7 auf dem Klettverschluss 82 der Klettverschlusselemente 8 ein Zwischenstück 10 vorgesehen ist, das auf der Oberseite mit einem Klettverschlussbereich 15 versehen ist, der korrespondierend zu dem Klettverschluss 82 der Klettverschlusselemente 8 ausgebildet ist, so dass das Längsgewölbeformteil 7 auf dem Zwischenstück 10 sicher gehalten werden kann. Auf der Unterseite des Zwischenstücks 10 ist ein zur Unterseite des Längsgewölbeformteils 7 korrespondierendes, nicht zu erkennendes Klettverschlusselement angeordnet, um eine Fixierung des Zwischenteils 19 auf den Klettverschlusselementen 82 in der Fersenkomponente zu ermöglichen.

In der Figur 6 ist auch detaillierter dargestellt, dass die Befestigungselemente 5 Hakenbereiche 55 aufweisen können, um über den übrigen Flauschbereich der Befestigungsbänder eine Festlegung an der Entlastungsorthese zu ermöglichen. Ebenfalls ist zu erkennen, dass die Y-Klettverschlussbänder 6 mit Hakenbereichen 66 zum Anlegen an Flauschabschnitte der Befestigungselemente 5, 6 versehen sind. Diese Ausgestaltungsform mit den Hakenbereichen 66, 55 kann allen Ausführungsbeispielen der vorgenannten Figuren ebenfalls ausgebildet sein.

## Patentansprüche

1. Entlastungsorthese mit einem Fußteil (2), das eine Sohle (3) aufweist, einem sich in proximaler Richtung von dem Fußteil (2) weg erstreckenden Unterschenkelteil (4) und zumindest einem Befestigungselement (5, 6) zur Festlegung der Orthese an einem Nutzer, das an dem Fußteil (2) und/oder dem Unterschenkelteil (4) angeordnet ist, wobei das Fußteil (2) längenveränderbar ausgebildet ist, **dadurch gekennzeichnet, dass** das Fußteil (2) eine Vorderfußkomponente (21) aufweist, die biegeelastisch ausgebildet ist.

2. Entlastungsorthese nach Anspruch 1, **dadurch gekennzeichnet, dass** das Fußteil (2) die Vorderfußkomponente (21) und eine Fersenkomponente (22) aufweist, die verschieblich zueinander und aneinander festlegbar ausgebildet sind.

3. Entlastungsorthese nach Anspruch 1 oder 2, **dadurch gekennzeichnet, dass** die Vorderfußkomponente (21) und die Fersenkomponente (22) in einer Längsführung (23, 24) aneinander gelagert und über Klemmelemente oder Formschlusselemente (9) zueinander fixierbar sind.

4. Entlastungsorthese nach einem der voranstehenden Ansprüche, **dadurch gekennzeichnet, dass** die Vorderfußkomponente (21) und die Fersenkomponente (22) Rastelemente und/oder Ausrichtmarkierungen (25) aufweisen, um die Komponenten (21, 22) zueinander auszurichten.

5. Entlastungsorthese nach einem der voranstehenden Ansprüche, **dadurch gekennzeichnet, dass** die Befestigungselemente (5, 6) verlagerbar an dem Fußteil (2) und/oder dem Unterschenkelteil (4) festgelegt sind.

6. Entlastungsorthese nach einem der voranstehenden Ansprüche, **dadurch gekennzeichnet, dass** die Befestigungselemente (5, 6) an Langlöchern (51) oder in diskreten Abständen zueinander angeordneten Ausnehmungen (61) festgelegt sind.

7. Entlastungsorthese nach einem der voranstehenden Ansprüche, **dadurch gekennzeichnet, dass** Klettverschlusselemente (8) in mehreren Positionen an dem Fußteil (2) festlegbar sind.

8. Entlastungsorthese nach einem der voranstehenden Ansprüche, **gekennzeichnet durch** ein Längsgewölbeformteil (7), das an dem Fußteil (2) befestigt ist und ein Fersenbein beim Gehen oder Stehen entlastet.

9. Entlastungsorthese nach Anspruch 8, **dadurch gekennzeichnet, dass** das Längsgewölbeformteil (7) lösbar oder verlagerbar an dem Fußteil (2) befestigt ist.

10. Entlastungsorthese nach Anspruch 8 oder 9, **dadurch gekennzeichnet, dass** das Längsgewölbeformteil (7) über zumindest eine Klettverschlussverbindung (71, 82), eine Längsführung oder in Abständen zueinander angeordnete Bohrungen oder Gewinde an dem Fußteil (2) befestigt ist.

11. Entlastungsorthese nach einem der Ansprüche 8 bis 10, **dadurch gekennzeichnet, dass** in oder an dem Längsgewölbeformteil (7) ein Röntgenorientierungspunkt angeordnet ist.

12. Entlastungsorthese nach einem der voranstehenden Ansprüche, **dadurch gekennzeichnet, dass** die Befestigungselemente (5, 6) als Y-Klettband ausgebildet sind.

13. Entlastungsorthese nach einem der voranstehenden Ansprüche, **dadurch gekennzeichnet, dass** sie als Fersenentlastungsorthese ausgebildet ist.

## Claims

1. Relief orthosis with a foot part (2), which has a sole (3), with a shank part (4), which extends away from the foot part (2) in the proximal direction, and with at least one fastening element (5, 6), which secures the orthosis on a user and which is arranged on the foot part (2) and/or on the shank part (4), wherein the foot part (2) is designed to be modifiable in length, **characterized in that** the foot part (2) comprises a forefoot component (21), which is flexurally elastic.

2. Relief orthosis according to claim 1, **characterized in that** the foot part (2) comprises the forefoot component (21) and a heel component (22), which are designed to be movable relative to each other and to be able to be secured on each other.

3. Relief orthosis according to claim 1 or 2, **characterized in that** the forefoot component (21) and the heel component (22) are mounted on each other in a longitudinal guide (23, 24) and can be fixed to each other via clamping elements or form-fit elements (9).

4. Relief orthosis according to one of the preceding claims, **characterized in that** the forefoot component (21) and the heel component (22) comprise latching elements and/or orientation markings (25), in order to adjust the components (21, 22) with respect to each other.

5. Relief orthosis according to one of the preceding claims, **characterized in that** the fastening elements (5, 6) are secured displaceable on the foot part (2) and/or on the shank part (4).

6. Relief orthosis according to one of the preceding claims, **characterized in that** the fastening elements (5, 6) are secured on oblong holes (51) or in recesses (61) arranged at discrete distances from one another.

7. Relief orthosis according to one of the preceding claims, **characterized in that** hook-and-loop fastener elements (8) can be secured in several positions on the foot part (2).

8. Relief orthosis according to one of the preceding claims, **characterized by** a longitudinal arch preform (7), which is secured on the foot part (2) and which relieves a calcaneus during walking or standing.

9. Relief orthosis according to claim 8, **characterized in that** the longitudinal arch preform (7) is secured detachable or displaceable on the foot part (2).

10. Relief orthosis according to claim 8 or 9, **characterized in that** the longitudinal arch preform (7) is secured on the foot part (2) via at least one hook-and-loop fastener connection (71, 82), a longitudinal guide or at distances from one another arranged bores or threads.

11. Relief orthosis according to one of the claims 8 to 10, **characterized in that** an X-ray orientation point is arranged in or on the longitudinal arch preform (7).

12. Relief orthosis according to one of the preceding claims, **characterized in that** the fastening elements (5, 6) are designed as a Y-shaped hook-and-loop tape.

13. Relief orthosis according to one of the preceding claims, **characterized in that** it is designed as a heel relief orthosis.

## Revendications

1. Orthèse de décharge comprenant une partie de pied (2) qui comporte une semelle (3), une partie de jambe (4) qui s'étend en direction proximale en éloignement de la partie de pied (2) et au moins un élément de fixation (5, 6) pour immobiliser l'orthèse sur un utilisateur, qui est agencé sur la partie de pied (2) et/ou sur la partie de jambe (4), dans laquelle la partie de pied (2) est réalisée à longueur variable,
**caractérisée en ce que** la partie de pied (2) comprend un composant d'avant-pied (21), qui est réalisé élastique en flexion.

2. Orthèse de décharge selon la revendication 1, **caractérisée en ce que** la partie de pied (2) comprend le composant d'avant-pied (21) et un composant de talon (22), lesquels sont réalisés de manière à pouvoir se déplacer l'un par rapport à l'autre et à être immobilisés l'un sur l'autre.

3. Orthèse de décharge selon la revendication 1 ou 2, **caractérisée en ce que** le composant d'avant-pied (21) et le composant de talon (22) sont montés l'un sur l'autre dans un guidage longitudinal (23, 24) et susceptibles d'être fixés l'un par rapport à l'autre via des éléments de serrage ou des éléments à coopération de formes (9).

4. Orthèse de décharge selon l'une des revendications précédentes, **caractérisée en ce que** le composant d'avant-pied (21) et le composant de talon (22) comprennent des éléments d'enclenchement et/ou des marques d'orientation (25), afin d'orienter les composants (21, 22) l'un par rapport à l'autre.

5. Orthèse de décharge selon l'une des revendications précédentes, **caractérisée en ce que** les éléments de fixation sont immobilisés de façon déplaçable sur la partie de pied (2) et/ou sur la partie de jambe (4).

6. Orthèse de décharge selon l'une des revendications précédentes, **caractérisée en ce que** les éléments de fixation (5, 6) sont immobilisés dans des trous oblongs (51) ou dans des évidements (61) agencés à des distances discrètes les uns par rapport aux autres.

7. Orthèse de décharge selon l'une des revendications précédentes, **caractérisée en ce que** des éléments de fermeture agrippante (8) sont susceptibles d'être immobilisés dans plusieurs positions sur la partie de pied (2).

8. Orthèse de décharge selon l'une des revendications précédentes, **caractérisée par** une pièce conformée à bombage longitudinal (7), qui est fixée sur la partie de pied (2) et qui décharge le calcanéum lors de la marche ou de la station debout.

9. Orthèse de décharge selon la revendication 8, **caractérisée en ce que** la pièce conformée à bombage longitudinal (7) est fixée de façon détachable ou déplaçable sur la partie de pied (2).

10. Orthèse de décharge selon la revendication 8 ou 9, **caractérisée en ce que** la pièce conformée à bombage longitudinal (7) est fixée sur la partie de pied (2) via au moins une liaison à fermeture agrippante (71, 82), un guidage longitudinal ou des perçages ou des vissages agencés à distance les uns des autres.

11. Orthèse de décharge selon l'une des revendications 8 à 10, **caractérisée en ce qu'**un point d'orientation de radiologie est agencé dans ou sur la pièce conformée à bombage longitudinal (7).

12. Orthèse de décharge selon l'une des revendications précédentes, **caractérisée en ce que** les éléments de fixation (5, 6) sont réalisés sous forme de bandes agrippantes de type Y.

13. Orthèse de décharge selon l'une des revendications précédentes, **caractérisée en ce qu'**elle est réalisée sous forme d'orthèse de décharge de talon.
